# EUROPEAN PATENT SPECIFICATION

(11) **EP 0 644 168 B1**
(45) Date of publication and mention of the grant of the patent: **15.07.1998**
(21) Application number: 94306660.5
(22) Date of filing: 12.09.1994
(51) Int. Cl.: C07C 2/72, B01J 23/04

(54) **Method for producing monoalkenyl benzenes**
Verfahren zur Herstellung von Monoalkenylbenzolen
Procédé pour la préparation de monoalkénylebenzènes

(30) Priority: 13.09.1993 JP 227366/93
(43) Date of publication of application: 22.03.1995
(73) Proprietor: NIPPON OIL CO. LTD., Minato-ku Tokyo (JP)
(72) Inventor: Matsuno, Mitsuo, Yokohama-shi, Kanagawa-ken (JP)
(74) Representative: Hallybone, Huw George

(56) References cited:
- EP-A- 0 128 001
- EP-A- 0 612 706
- STN-INFORMATION SERVICE; FILE :CA ; AN: 119:138841 'Preparation of alkenylbenzenes as intermediates for naphthalenedicarboxylic acids'
- STN-INFORMATION SERVICE; FILE: CA AN: 119:138842 'Preparation of alkenylbenzene derivatives'

## Description

This invention relates to a method for producing monoalkenyl benzene. More particularly, it relates to a method for producing monoalkenyl benzene employed as a precursor for pharmaceuticals, agricultural chemicals or high molecular materials.

There has hitherto been known a method for producing monoalkenyl benzene by reacting alkyl benzene with a conjugated diene, such as 1,3-butadiene, in the presence of an alkali metal catalyst (German Patent 557514). However, this known method has a yield of 30% at most and hence is inconvenient for practicing industrially.

There has also been proposed a method in which, for improving the above method, an oxide of an alkali metal or an alkaline earth metal, such as calcium oxide, is employed as a catalyst (Us Pat. No. 3244758). However, the catalyst has a prolonged induction period and a lot of time is required until the maximum activity is achieved, that is until a steady state of the reaction is reached, thus raising problems in connection with economic profitability and operational safety. In addition, since with these methods not only monoalkenyl benzene, but also compounds in which conjugated diene is further added to monoalkenyl benzene or higher molecular compounds are produced simultaneously, these polymers covering the catalyst surface gradually deactivate the catalyst. Besides, such catalyst tends to be lowered not only in activity but also in selectivity. As resinous polymers made by such catalyst tend to encompass the catalyst per se to solidify same, the catalyst is not completely deactivated and still contains active parts so that when the used catalyst is extracted for exchange, it is likely to be ignited due to contact with oxygen and moisture in the atmosphere to cause fire, thus raising difficulties in handling.

JP-A-05097722 describes methods for producing alkenylbenzenes by treating alkyl benzenes with 1,3-butadiene substantially in the absence of oxygen and water, and in the presence of catalysts prepared by dispersing sodium on inorganic compositions containing calcined and dehydrated KOH. The catalyst is prepared by calcining KOH at 450°C for 3 hours, supported on alumina, followed by drying at 400 to 450°C for 5 hours, dispersion in o-xylene, and mixing with a dispersion of sodium in o-xylene at 140°C for 1 hour.

JP-A-05112476 describes methods for the preparation of alkenylbenzenes derivatives by treating alkyl benzenes with conjugated diolefins in the absence of oxygen and water, using catalysts prepared by dispersing alkyl metals on a potassium aluminate substrate. The potassium aluminate substrate is prepared by evaporating an aqueous solution of potassium and aluminum hydroxides in water, followed by dehydration at 200-450°C for 3 to 10 hours.

EP-A-0612706, which forms part of the state of the art by virtue of Article 54(3) EPC, describes a process for producing monoalkenylbenzenes by treating alkyl benzenes with a conjugated diene in the presence of a catalyst produced by calcining a mixture of a basic potassium compound and alumina at 500-700°C, followed by heat treating the calcined product together with metallic sodium in an atmosphere of an inert gas. According to this reference, a calcination temperature higher than 700°C results in insufficient catalytic activity because of insufficient dispersion of metallic sodium on the potassium aluminate substrate.

EP-A-0128001 describes a process for preparing alkyl benzenes by treating aromatic hydrocarbons with mono-olefins in the presence of a catalyst comprising sodium and/or sodium amide dispersed on a carrier represented by the formula K₂O·xAl₂O₃ where x is 0.5 ≤ x ≤ 11.

It is an object of the present invention to provide a method for producing monoalkenylbenzenes capable of producing an object compound in reduced induction period with high selectivity and high yield.

The above and other objects of the invention will become apparent from the following description.

According to the present invention, there is provided a method for producing a monoalkenyl benzene comprising reacting an alkyl benzene having carbon atoms of not more than 10 with a conjugated diene compound having carbon atoms of 4 to 6 in the presence of a catalyst containing a carrier represented by the formula of

K₂O·xAl₂O₃ where x is 0.5 ≤ x ≤ 11,

and a catalyst component selected from the group consisting of sodium, sodium amide and mixtures thereof carried on the carrier, wherein the carrier is produced by mixing a potassium-containing compound with an aluminium-containing compound and reacting the resulting mixture at 900 to 2000°C.

The present invention will be explained hereinbelow in detail.

The catalyst employed in the method of the present invention is produced by having sodium and/or sodium amide carried on a specified carrier. The amount of sodium and/or sodium amide carried by the carrier is preferably 0.1 to 20 wt% and preferably 1 to 15 wt% as sodium atoms, based on the weight of the carrier. Even though the catalyst of the present invention contains sodium and/or sodium amide in an amount as large as 20 wt% as sodium atom, the catalyst is dry and exhibits good dispersibility and high activity, whereas, if the amount of the catalyst component is lowered, the catalyst is not lowered in selectivity at all, although the activity of the catalyst is slightly lowered. By-products, such as tar or resinous products are hardly produced by the catalyst. Since the carried amount of the catalyst component can be increased, the catalyst exhibits strong resistance against impurities brought into the reaction system, and is capable of maintaining high activity and selectivity for an extremely long time period. Although the structure of such catalyst is not absolutely clear, it is considered that a part of sodium atoms is physically and chemically adsorbed to the catalyst surface, and also a part of sodium atoms is substituted for atoms constituting the carrier.

In the present invention, the carrier constituting the catalyst is represented by the formula K₂O·xAl₂O₃, where x is. 0.5 ≤ x ≤ 11 and preferably 0.7 ≤ x ≤ 10. There is no particular limitation to the shape and size of the carrier which may be suitably selected depending on the type or volume of the reactor or reaction conditions. The catalyst is usually used as powders, pellets or particles and, more specifically, may have a particle size of 0.1 mm to 10 mm. The above formula for the carrier indicates that the carrier is basically made up of K₂O and Al₂O₃ as its components and represents, for convenience sake, the composition of a carrier in case of changes in the charging composition of the carrier materials as later explained. That is, the above formula does not mean that K₂O and Al₂O₃ remain as such in the carrier but are present in the double oxide form. Consequently, the above-mentioned carrier is not a simple mixture of K₂O and Al₂O₃ and the favorable effects of the present invention cannot be achieved with the use of such simple mixture.

Although there is no limitation to the method for producing the carrier, it may be produced by mixing at least one potassium-containing compound with at least one aluminum-containing compound together and reacting them under elevated temperatures.

Although there is no limitation to the potassium-containing compound, provided that such compound may be reacted at elevated temperatures to produce potassium oxides. Thus the potassium-containing compound may be enumerated by, for example, potassium hydroxide, potassium hydrogen carbonate, potassium carbonate, potassium hydride and potassium acetate and a compound represented by the formula KOR¹ or KR², where R¹ and R² represent a linear or branched aliphatic hydrocarbon residue with 1 to 20 and preferably 1 to 8 carbon atoms, or an aryl or aralkyl group with 6 to 30 and preferably 6 to 12 carbon atoms. Specifically, R¹ and R² include alkyl groups, such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, dodecyl or hexadecyl groups; alkenyl groups, such as vinyl or allyl groups; aryl groups, such as phenyl, tolyl, xylyl, cumenyl, or triphenyl groups; and aralkyl groups, such as benzyl, dimethyl benzyl or phenylethyl groups. The potassium-containing compounds, represented by the formula KOR¹ or the formula KR², preferably include methylpotassium, vinylpotassium, ethylpotassium, phenylpotassium, tolylpotassium, benzylpotassium, dodecylpotassium, hexadecylpotassium, triphenylpotassium, dimethyl benzylpotassium, phenyl ethylpotassium, ethoxypotassium, butoxypotassium and isopropoxypotassium. These potassium-containing compounds may contain hydrates and may be used singly or in combination.

The aluminum-containing compounds include alumina hydrates, such as hydrargillite, nordstrandite, bayerite, boehmite or diaspore; aluminas such as α-alumina, Θ-alumina, κ-alumina, δ-alumina, η-alumina, γ-alumina, χ-alumina, or ρ-alumina; and an aluminum-containing compound represented by the formula Al(OR³)₃, where R³ denotes a linear or branched aliphatic hydrocarbon group having 1 to 20 and preferably 1 to 8 carbon atoms or an aryl or aralkyl group having 6 to 30 and preferably 6 to 12 carbon atoms and may preferably be enumerated by alkyl, aryl and aralkyl groups specifically enumerated in connection with R¹ and R². The aluminum-containing compounds represented by the formula Al(OR³)₃ specifically include, for example, trimethoxy aluminum, triethoxy aluminum, tripropoxy aluminum, triisopropoxy aluminum, tributoxy aluminum, triisobutoxy aluminum, tri-sec-butoxy aluminum, tri-tert-butoxy aluminum, tripentyloxy aluminum, triisopentyloxy aluminum, trineopentyloxy aluminum, tri-tert-pentyloxy aluminum, trihexyloxy aluminum, triisohexyloxy aluminum, triheptyloxy aluminum, trioctyloxy aluminum, triphenoxy aluminum and tribenzyloxy aluminum.

For reacting the potassium-containing compound with the aluminum-containing compound, preferably the two kinds of compounds are mixed so that the K/Al ratio is equal to a preset value of x in the formula K₂O·xAl₂O₃, and the reaction is carried out in the presence or absence of air, nitrogen or the like, at a reaction temperature of 900 to 2000°C and for 1 to 20 hours, more preferably at 900 to 1500°C for 2 to 10 hours. The resulting carrier is classified to the desired particle size by crushing and sifting or by granulation and calcination.

For producing the catalyst by having sodium and/or sodium amide, for example, sodium supported on the carrier, there may be employed a method comprising agitating and mixing the carrier and sodium for 30 minutes to 10 hours in the absence of a solvent at a temperature not lower than the melting point of sodium, preferably at a temperature of 120 to 400°C, a method comprising depositing sodium vapor on the carrier by known methods, and a method comprising agitating sodium and the carrier at a high speed in a high-boiling solvent, such as white oil, at a temperature exceeding the melting point of sodium, preferably at 120 to 400°C. When sodium amide is supported on the carrier, sodium is dissolved in liquid ammonia to form an ammonia solution of sodium amide, in which the carrier is immersed at a temperature preferably 0 to 200°C for a sufficiently long time, usually 30 minutes to 10 hours, after which ammonia is vaporized off. If sodium and sodium amide are supported on the carrier, one or more of the above methods may be selected and combined in any suitable manner.

The catalysts employed in the method according to the present invention may be employed directly or after previous hydrogenating treatment. Such hydrogenation treatment may be carried out by contacting hydrogen and the catalyst at 0 to 400°C under an atmospheric pressure to 100 kg/cm² for 30 minutes to 10 hours. Such hydrogen treatment improves the effect of further shortening the induction period.

The above-mentioned catalysts have properties free from coagulation due to improved dispersibility, high activity and selectivity and require substantially no induction period before starting the reaction, so that the catalysts may be suitable not only for fixed bed reactors but also continuous stirred tank reactors in which the catalyst is continuously introduced along with the starting material into the reactor.

With the method of the present invention, alkyl benzene having 10 or less carbon atoms is reacted with a conjugated diene compound with 4 to 6 carbon atoms, using the above catalyst for producing monoalkenyl benzene.

Alkyl benzene is a compound produced by substituting hydrogen atoms on a benzene ring for at least one and preferably 1 to 4 alkyl groups. The alkyl groups may be enumerated by, for example, methyl or ethyl groups.

Alkyl benzene includes ortho-, meta- and para-substituted ones. Specifically, toluene, ethyl benzene, xylene, diethyl benzene, trimethyl benzene, ethyl dimethyl benzene and tetramethyl benzene, may be employed in the method of the present invention. Of these, o-xylene, m-xylene and p-xylene are preferred. Two or more of these compounds may also be employed as a mixture.

The above-mentioned conjugated diene compound includes, for example, 1,3-butadiene, isoprene, 1,3-pentadiene, 2,3-dimethyl butadiene, 1,3-hexadiene and 2,4-hexadiene.

The alkyl benzene and conjugated diene compound are not required to be of high purity. Thus it is only sufficient if compounds other than the object compound, such as olefin, diolefin, alkyl benzene, water or a carbon dioxide gas, are removed to the industrially feasible usual level.

The reaction between alkyl benzene and the conjugated diene compound in the presence of the above-mentioned catalyst is the reaction of alkenylation which may be carried out by a variety of types of the reaction. Thus the reaction may be carried out by a batch or semi-batch type reaction employing an autoclave, by a continuous reaction method in which the catalyst and the starting material, that is alkyl benzene and the conjugated diene compound, are continuously supplied to an autoclave for a continuous stirred tank reactor system, and by a continuous reaction method employing a fixed bed in which the catalyst is charged into a reactor through which alkyl benzene and the conjugated diene compound are supplied.

Although the alkenylation reaction conditions are suitably selected depending on the amount of the catalyst in use and the charging amount, the reaction temperature is preferably 80 to 200°C and more preferably 100 to 180°C. The reaction time for the batch or semi-batch type reaction or the residence time for the continuous reaction method is preferably 5 minutes to ten hours and more preferably 10 minutes to five hours. If the continuous reaction method employing a fixed bed is employed, the liquid hourly space velocity (LHSV) is preferably 0.1 to 10 (V/V·hr). The pressure during the reaction is preferably 5 to 200 kgf/cm² and more preferably 10 to 100 kgf/cm².

The charging molar ratio of alkyl benzene to the conjugated diene compound in the alkenylation reaction is preferably 1:0.001 to 1:0.5 and more preferably 1:0.01 to 1:0.4 mol. Although there is no limitation to the amount of the catalyst in use, the amount of 0.5 to 20 wt% and, above all, 1 to 15 wt% of the catalyst based on the weight of alkyl benzene, is preferred. The amount of the catalyst in use herein means the amount of the carrier, sodium and/or sodium amide taken together.

In the alkenylation reaction, solvents may be employed insofar as they are not harmful to the reaction. These solvents may be enumerated by inert hydrocarbon compounds, such as hexane, heptane or benzene.

In the production method of the present invention, unreacted substances may be removed by distillation or trapping after the end of the alkenylation reaction for producing the objective monoalkenyl benzene at a high yield.

By employing the specified catalyst in accordance with the present invention, a larger amount of sodium and/or sodium amide may be carried on the carrier than on the conventional catalyst, thereby improving activity and selectivity and allowing to produce monoalkenyl benzene, employed as a precursor for pharmaceuticals, agricultural chemicals and high molecular materials, with high reaction rate and yield.

The present invention will now be described with reference to Examples and Comparative Examples. These embodiments are, however, given only by way of illustration and are not intended for limiting the invention.

### Example 1

Fine powders produced by crushing 66 g of pellets of potassium hydroxide containing 15 wt% of moisture, and 60 g of boehmite were mixed thoroughly. The resulting mixture was charged into an alumina crucible and calcined in an air atmosphere at 1200°C for five hours. After cooling, the calcined product was taken out and pulverized in a centrifugal ball mill for two hours to give a powdered product having a size less than 60 mesh size which was used as a carrier (K₂O·0.98Al₂O₃). 60 g of the carrier were charged into a three-necked flask of 300 ml capacity and heated to 150°C under a nitrogen atmosphere. To the heated mass were added 6 g of sodium under agitation. After the end of the addition, the temperature was raised to and maintained at 200°C and agitation was continued for one hour to have sodium supported on the carrier uniformly for preparing the catalyst.

After a stainless steel autoclave with magnetic stirrer of 1000 ml capacity had its atmosphere thoroughly replaced by nitrogen, 16 g of the catalyst, 425 g of o-xylene and 54 g of 1,3-butadiene were introduced into the autoclave and reacted at 150°C for two hours. After the reaction, the reaction system was cooled to 100°C and butadiene which remained unreacted was collected by a trap in a dry ice-methanol bath. Unreacted o-xylene and reaction products remaining in the reactor were recovered by vacuum distillation. 5 g of butadiene were recovered, while 470 g of a liquid product were recovered by vacuum distillation. Analysis by gas chromatography revealed that 131 g of 5-(o-tolyl) pentene-2 were produced. The yield was 82%.

### Example 2

54 g of 1,3-butadiene and 425 g of o-xylene were charged into the autoclave in a reduced-pressure state employed in Example 1 and reaction was continued at 150°C for one hour. The reaction was discontinued since a pressure gauge of the autoclave indicated zero atmosphere and the residual butadiene quantity was determined to be substantially zero. Post-treatment was carried out in the same way as in Example 1. 0.6 g of butadiene and 475 g of a liquid product were recovered, while 142 g of 5-(o-tolyl) pentene-2 were produced. The yield was 88.6%.

### Example 3

Reaction was carried out in the same way as in Example 1 except employing p-xylene in place of o-xylene. As a result, 5-(p-tolyl) pentene-2 was obtained with a yield of 78.3%.

### Example 4

Reaction was carried out in the same way as in Example 1, except employing m-diethylbenzene in place of o-xylene and setting the scale for the catalyst and the reagents to 1/10. 5-(3-ethylphenyl) hexene-2 was produced with a yield of 64.1%.

### Example 5

Reaction was carried out in the same way as in Example 1, except employing 75 g of 1,3-pentadiene (purity, 90%; cis/trans ratio = 23.4/76.6) in place of 54 g of 1,3-butadiene. A mixture of 1-(o-tolyl) hexene-3 and 4-methyl-5-(o-tolyl) pentene-2 was produced with a yield of 67.7%.

### Example 6

Reaction was carried out in the same way as in Example 1, except employing 15 g of a catalyst comprised of the carrier similar to that used in Example 1 and 2 wt% of sodium supported on the carrier. As a result, 5-o-(tolyl) pentene-2 was produced with a yield of 49%.

### Example 7

Reaction was carried out in the same way as in Example 1, except employing 15 g of a catalyst comprised of the carrier similar to that used in Example 1 and 20 wt% of sodium supported on the carrier. As a result, 5-o-(tolyl) pentene-2 was produced with a yield of 93%.

### Example 8

A carrier (K₂0·1.1Al₂O₃) was prepared in the same way as in Example 1, except using 234 g of aluminum hydroxide (manufactured by WAKO PURE CHEMICAL INDUSTRIES, LTD.) and 180 g of potassium hydroxide as starting materials. To 60 g of the produced catalyst were added 6 g of sodium under heating at 200 to 220°C using a device similar to Example 1. After the end of the addition, the resulting mixture was agitated for three hours to have sodium supported on the carrier for preparing the catalyst. The produced catalyst was in the form of dark blue-colored powders exhibiting satisfactory dispersibility.

Reaction was carried out in the same way as in Example 1, except employing the above-mentioned catalyst and setting the reaction temperature to 140°C. As a result, 5-(o-tolyl) pentene-2 was produced with a yield of 71%.

### Example 9

138 g of anhydrous potassium carbonate and 78 g of aluminum hydroxide are adjusted to have a uniform particle size each of smaller than 16 meshes and mixed together sufficiently uniformly. The resulting mixture was sintered at 1200°C for five hours to prepare a carrier (K₂O·0.5Al₂O₃). 15 g of the produced carrier and 1.5 g of sodium were charged into a stainless steel autoclave, into which 30 g of liquid ammonia were introduced under pressure and reaction was carried out at room temperature under agitation for two hours. After the end of the reaction, hydrogen yielded by the reaction between ammonia and sodium and ammonia was released to the atmosphere. Alkenylation reaction was carried out in the same way as in Example 1, except employing the catalyst prepared as described above. 5-(o-tolyl) pentene-2 was produced with a yield of 78%.

### Example 10

70 g of anhydrous potassium carbonate and 660 g of boehmite were sintered at 1800°C for seven hours to produce a carrier represented by the formula (K₂O·10.7Al₂O₃). The diffraction X-ray method revealed that no alumina was present in the carrier. A catalyst was prepared by having 2 wt% of sodium supported on the produced carrier in the same manner as in Example 1.

17 g of the catalyst were charged into an autoclave, into which 212 g of o-xylene and 54 g of butadiene were added and reaction was carried out at 160°C for two hours. As a result, 5-(o-tolyl) pentene-2 was produced with a yield of 45%.

### Example 11

Aluminum hydroxide and potassium hydroxide were weighed at the same ratio as in Example 8. To the resulting mixture was added a minor amount of water and kneaded together. The resulting mass was extruded to give an extruded product having a diameter of 1.6 mm by a molding machine. The resulting molded product was dried at 150°C for one hour and sintered at 900°C for eight hours. The resulting sintered product was fractured to pieces each about 3 to 5 mm in length so as to be used as a carrier. To the resulting carrier was added sodium at 200°C under a nitrogen atmosphere so that sodium in the catalyst accounted for 10 wt%. A catalyst was prepared under agitation slowly for about two hours.

30 g of the above-mentioned catalyst were charged under a nitrogen atmosphere into a center segment of a reaction tube of stainless steel of a fixed bed reactor 30 mm in inside diameter and 500 mm in length. o-Xylene and butadiene were charged into a tank for starting materials at a molar ratio of 10:1. A starting material mixture was then introduced by a pump at a liquid hourly space velocity (LHSV) of 1.0 hr⁻¹ for reaction at 140°C under 20 kgf/cm². The reaction rate of butadiene reached 68% in three hours after start of the reaction. The half-value period of catalytic activity, that is the time until the reaction rate of butadiene is decreased by one-half from its maximum value, was 1500 hours or more. The reaction liquid recovered was freed of unreacted butadiene and subsequently o-xylene was recovered in a distillation column for re-use. 5-(o-tolyl) pentene-2 was recovered under vacuum distillation. It was found that there was substantially no bottom residue in the distillation column and it was shown that high-boiling products were hardly produced.

### Example 12

200 g of potassium hydrogen carbonate and 202 g of γ-alumina were sufficiently mixed and sintered at 1000°C for seven hours to give a carrier (K₂O·Al₂O₃). 1.5 g of sodium were added to 15 g of the produced carrier and agitated at 200°C for one hour to have sodium supported on the carrier.

The produced catalyst was charged in its entirety into a stainless steel autoclave of 1 lit. capacity. 100 ml of n-heptane as a solvent was charged into the autoclave and the temperature was raised to 160°C. Pressure was raised with hydrogen to 70 kgf/cm² and agitation was carried out for three hours. Pressure decrease during this time was 2.3 kgf/cm². After cooling, residual hydrogen was discharged. 212 g of o-xylene and 27 g of butadiene were introduced under pressure into the autoclave and reaction was carried out at 150°C for three hours. As a result, 5-(o-tolyl) pentene-2 was produced with a yield of 62%.

### Example 13

112 g of t-butoxy potassium and 246 g of aluminum-sec-butoxide were charged into 200 ml of t-butanol and mixing was carried out at 70°C under a nitrogen atmosphere. K[Al(OC(CH₃)₃)(OCH(CH₃)CH₂CH₃)₃], an ate-complex, was precipitated as a white-tinted precipitate. After the solvent t-butanol was distilled off under vacuum, the resulting precipitate was pre-sintered in a nitrogen stream for four hours at 500°C for decomposing the organic residues in their entirety. The temperature was raised to 1200°C and sintering was further continued for three hours to produce a carrier (K₂O·Al₂O₃). To 15 g of the produced carrier were added 1.5 g of sodium under a nitrogen stream and supported on the carrier by agitation at 200°C for two hours to produce a catalyst.

Reaction was carried out in the same way as in Example 1, except employing the total quantity of the above catalyst. As a result, 5-(o-tolyl) pentene-2 was produced with a yield of 86%.

### Comparative Example 1

To 15 g of potassium carbonate dried at 500°C for five hours, 3 g of sodium were added under nitrogen atmosphere, and the resulting mass was agitated vigorously at 200°C in order to have 20 wt% of sodium supported on the carrier for producing a catalyst. The produced catalyst, which was pasty in its entirety at 200°C, was solidified in the form of wax in its entirety when allowed to cool to room temperature.

Reaction was carried out in the same way as in Example 1, except employing 16 g of the above catalyst. As a result, 5-(o-tolyl) pentene-2 was produced with a yield of 8%.

### Comparative Example 2

2 wt% of sodium was supported on the carrier in the same way as in Comparative Example 1 for preparing a catalyst. In distinction from the Comparative Example 1, the catalyst produced was purplish in color and excellent in dispersibility.

Reaction was carried out in the same way as in Example 1, except employing 15 g of the above catalyst. As a result, 5-(o-tolyl) pentene-2 was produced with a yield of 27%.

Although the yield was improved by three times that of Comparative Example 1, it is about one half of that of Example 6, demonstrating that the inventive method is superior to the method of the Comparative Example.

### Comparative Example 3

Potassium carbonate admixed with 1 wt% of graphite as a binder was molded into a pellet about 3 mm in size, which was dried at 200°C for 15 hours to produce a carrier. 5 wt% of sodium was carried on the produced carrier under a nitrogen atmosphere to prepare a catalyst.

Continuous reaction was carried out in the same way as in Example 11 except using the above catalyst. As a result, 95 hours were required until a maximum value of reactivity was exhibited. 5-(o-tolyl) pentene-2 was produced with a yield of 29%. The half-value period of catalytic activity was 400 hours.

The catalyst thus prepared differed from that of Example 11 in the catalyst shape and the carried amount of sodium which was one-half of that of Example 11. The catalyst in the present Comparative Example suffered from a longer induction period, a faster rate of the lowering of catalytic activity and a lower yield of 5-(o-tolyl) pentene-2. It was apparent that the inventive catalyst also had properties superior to those of the conventional catalyst for such continuous reaction.

### Comparative Example 4

100 g of activated charcoal and 100 g of potassium hydroxide (containing 15 wt% of moisture) were mixed thoroughly by a ball mill and dried at 150°C for five hours to prepare a catalyst. 3 g of sodium was supported on 15 g of the produced carrier at 150°C under a nitrogen atmosphere. A catalyst with superior dispersibility was prepared.

Reaction was carried out in the same way as in Example 1, except employing 15 g of the above-mentioned catalyst. As a result, 5-(o-tolyl) pentene-2 was produced with a yield of 2%. o-Xylene, a starting material, accounted for a major portion of the recovered product. After injecting methanol into the reactor for deactivating the catalyst, the reactor was opened and checked. It was found that a viscous material was left in the reactor. As a result of analyses, this material was identified as being a low butadiene polymer.

## Claims

1. A method for producing a monoalkenyl benzene comprising reacting an alkyl benzene having carbon atoms of not more than 10 with a conjugated diene compound having carbon atoms of 4 to 6 in the presence of a catalyst containing a carrier represented by the formula of
K₂O·xAl₂O₃ where x is 0.5 ≤ x ≤ 11,
and a catalyst component selected from sodium, sodium amide and mixtures thereof carried on said carrier, wherein the carrier is produced by mixing a potassium-containing compound with an aluminium-containing compound and reacting the resulting mixture at 900 to 2000°C.

2. The method as claimed in Claim 1 wherein 0.1 to 20 wt% of the catalyst component is contained as sodium atoms based on a weight of the carrier.

3. The method as claimed in Claim 1 or 2 wherein the step of reacting the resulting mixture is carried out for 1 to 20 hours.

4. The method as claimed in any preceding Claim wherein the catalyst is hydrogenated prior to reacting said alkyl benzene with said conjugated diene compound.

5. The method as claimed in Claim 4 wherein the catalyst is contacted with hydrogen at 0 to 400°C at a pressure from atmospheric pressure to 100 kg/cm² for 30 minutes to 10 hours.

6. The method as claimed in any preceding Claim wherein said catalyst is contained in an amount of 0.5 to 20 wt% based on a weight of the alkyl benzene.

## Patentansprüche

1. Verfahren zur Herstellug von Monoalkenylbenzolen mittels Umsetzung eines Alkylbenzols mit nicht mehr als 10 Kohlenstoffatomen mit einer konjugierten Dienverbindung mit 4 bis 6 Kohlenstoffatomen unter Anwesenheit eines Katalysators mit einem Träger der Formel
K₂O·xAl₂O₃, wobei 0,5 ≤ x ≤ 11,
und einem Katalysatorbestandteil aus Natrium oder Natriumamid oder einer Mischung daraus, die auf dem Träger gebunden ist, wobei der Träger durch Mischen einer kaliumhaltigen Verbindung mit einer aluminiumhaltigen Verbindung und Reaktion der erhaltenen Mischung bei 900 bis 2000°C erzeugt wird.

2. Verfahren nach Anspruch 1, bei dem 0,1 bis 20 Gew.-% des Katalysatorbestandteils als Natriumatomen enthalten sind, bezogen auf das Gewicht des Trägers.

3. Verfahren nach Anspruch 1, bei dem der Schritt der Reaktion der erhaltenen Mischung zwischen 1 und 20 Stunden lang durchgeführt wird.

4. Verfahren nach einem der vorhergehenden Ansprüche, bei dem der Katalysator vor der Umsetzung des Alkylbenzols mit der konjugierten Dienverbindung hydriert wird.

5. Verfahren nach Anspruch 4, bei dein der Katalysator mit Wasserstoff bei 0 bis 400°C unter einem Druck vom Normaldruck bis 100 kg/cm² zwischen 30 Minuten und 10 Stunden lang in Kontakt gebracht wird.

6. Verfahren nach einem der vorhergehenden Ansprüche, bei dem der Katalysator in einer Menge von 0,5 bis 20 Gew.-% bezogen auf das Gewicht an Alkylbenzol enthalten ist.

## Revendications

1. Procédé de production d'un monoalcénylbenzène, comprenant la mise à réagir d'un alkylbenzène n'ayant pas plus de 10 atomes de carbone avec un composé diène conjugué ayant de 4 à 6 atomes de carbone en présence d'un catalyseur contenant un support représenté par la formule
K₂O·xAl₂O₃ où 0,5 ≤ x ≤ 11,
et un composant catalyseur sélectionné parmi le sodium, l'amide de sodium ou leurs mélanges, porté sur ledit support, où le support est produit par mélange d'un composé contenant du potassium avec un composé contenant de l'aluminium et mise à réagir du mélange obtenu entre 900 et 2 000°C.

2. Procédé selon la revendication 1, où 0,1 à 20 % en poids du composant catalyseur sont présents sous forme d'atomes de sodium par rapport au poids du support.

3. Procédé selon la revendication 1 ou 2, ou l'étape de mise à réagir du mélange obtenu est mise en oeuvre pendant 1 à 20 h.

4. Procédé selon l'une quelconque des revendications précédentes, où le catalyseur est hydrogéné avant la mise à réagir dudit alkylbenzène avec ledit composé diène conjugué.

5. Procédé selon la revendication 4, où le catalyseur est mis en contact avec de l'hydrogène entre 0 et 400°C à une pression comprise entre la pression atmosphérique et 100 kg/cm² pendant 30 min à 10 h.

6. Procédé selon l'une quelconque des revendications précédentes, où ledit catalyseur est présent en une quantité de 0,5 à 20 % en poids par rapport au poids d'alkylbenzène.
